# EUROPÄISCHE PATENTSCHRIFT

(11) **EP 0 999 814 B1**
(45) Veröffentlichungstag und Bekanntmachung des Hinweises auf die Patenterteilung: **25.09.2002**
(21) Anmeldenummer: 98942542.6
(22) Anmeldetag: 13.07.1998
(51) Int. Cl.: A61F 13/56, A61F 13/58

(54) **VERFAHREN ZUM HERSTELLEN VON VERSCHLUSSELEMENTEN BEI SAUGFÄHIGEN HYGIENEARTIKELN ZUM EINMALIGEN GEBRAUCH**
METHOD FOR PRODUCING CLOSURE ELEMENTS FOR SINGLE USE ABSORBENT HYGIENE ARTICLES
PROCEDE POUR LA FABRICATION D'ELEMENTS DE FERMETURE DESTINES A DES ARTICLES D'HYGIENE ABSORBANTS A USAGE UNIQUE

(30) Priorität: 29.07.1997 DE 19732552
(43) Veröffentlichungstag der Anmeldung: 17.05.2000
(73) Patentinhaber: Paul Hartmann Aktiengesellschaft, 89522 Heidenheim (DE)
(72) Erfinder: MERKLE, Regina, D-73450 Neresheim (DE)
(74) Vertreter: Friz, Oliver
(86) Internationale Anmeldenummer: EP9804336
(87) Internationale Veröffentlichungsnummer: WO99006000

(56) Entgegenhaltungen:
- EP-A- 0 530 781
- US-A- 4 946 527
- US-A- 5 383 871

## Beschreibung

Die Erfindung betrifft ein Verfahren zum Herstellen von Verschlusselementen bei saugfähigen Hygieneartikeln zum einmaligen Gebrauch, insbesondere Windeln, Inkontinenzartikeln, Windel- oder Inkontinenzhöschen oder - vorlagen, wobei die mechanisch wirkenden Verschlusselemente durch Aufbringen eines Haftschmelzklebers klebend ausgestaltet werden.

Ein derartiges Verfahren ist aus US-A-4,946,527 bekannt.

Mechanisch wirkende Verschlusselemente, insbesondere als Teil eines Klettverschlusssystems sind vielfach bekannt.

Der vorliegenden Erfindung liegt die Aufgabe zugrunde, ein Verfahren der vorstehend erwähnten Art anzugeben, mit dem die Eigenschaften der mechanisch wirkenden Verschlusselemente besser vorgebbar bzw. den Anforderungen anpassbar sind.

Diese Aufgabe wird durch ein Verfahren mit den Merkmalen des Anspruchs 1 gelöst.

Die mechanisch wirkenden Verschlusselemente können demnach wenigstens teilweise aus einem klebenden Material hergestellt werden oder es kann wenigstens auf einen Teil ihrer Oberfläche ein permanent wirkender Haftkleberauftrag aufgebracht werden.

Wenn die mechanischen Verschlusselemente Teil eines Klettverschlusssystems sind, so kann es sich hierbei um die hintergreifende hakenbildende Komponente des Klettverschlusssystems handeln, während eine hintergreifbare schlaufenbildende Komponente von einem im weitesten Sinne flauschförmigen, insbesondere vliesförmigen Material, gebildet sein kann. Die mechanisch wirkenden Verschlusselemente können eine Tragschicht umfassen, von deren Oberfläche hakenbildende Vorsprünge vorstehen. Die hakenbildenden Vorsprünge umfassen einen in irgendeiner Weise hintergreifbaren Abschnitt. Sie umfassen beispielsweise einen Stamm und einen das von der Tragschicht abgewandte freie Ende bildenden Kopf, welcher den hintergreifbaren Abschnitt bildet.

Es wird solchenfalls auf zumindest einen Teil der Oberfläche der hakenbildenden Vorsprünge ein Haftkleber aufgetragen, so dass die Verschlusselemente, die hakenbildenden Vorsprünge sowohl mechanische als auch klebende Halte- oder Hafteigenschaften ausbilden können.

Das Haftklebermaterial kann auf zumindest einem Teil der von der Tragschicht abgewandten Oberfläche des Kopfs der hakenbildenden Vorsprünge aufgetragen werden. Es ist jedoch auch denkbar, dass der Haftkleber zudem auf der Oberfläche des Stamms aufgetragen wird. Zum Erreichen bestimmter Charakteristiken erweist es sich als vorteilhaft., wenn der Haftkleber zudem auf zumindest einem Teil der Oberfläche der Tragschicht zwischen den hakenbildenden Vorsprüngen aufgetragen wird.

Die mechanischen Verschlusselemente, die im einfachsten Fall stiftförmige Vorsprünge umfassen können bzw. die hakenbildenden Vorsprünge können in der Draufsicht in Form von zueinander parallelen Linien jeweils nebeneinander oder zueinander versetzt oder in sonstiger Weise angeordnet sein. Es erweist sich auch als vorteilhaft, wenn die mechanischen Verschlusselemente vor dem Auftragen eines Haftklebers einer Vorbehandlung unterzogen werden, beispielsweise einer Coronabehandlung, einer Plasmabeschichtung oder einer Beschichtung durch einen Haftvermittler (Primer).

Als Haftklebermaterialien lassen sich sog. Hotmelts, d.h. Haftschmelzkleber (PSA), z.B. auf Polyolefinbasis oder auf synthetischer Kautschukbasis, verwenden. Diese Materialien zeichnen sich durch eine sehr hohe Adhäsion und einer entsprechend gewählten variablen Kohäsion aus. Die Applikationstemperatur liegt im Bereich von 100 bis 180°C. Die Viskositäten sind im Bereich dieser Applikationstemperatur abhängig von der nachfolgend zu beschreibenden Applikationstechnik; sie betragen jedoch wenigstens 800 mPas und höchstens 15000 mPas.

Denkbar und vorteilhaft sind auch zum einen Dispersions-Haftkleber, bevorzugt auf Wasserbasis, und zum anderen Lösemittel-Haftkleber. Dispersions- und Lösemittelhaftkleber werden jeweils bevorzugt mit einem Feststoffanteil (Gew.-%) von über 50 % verarbeitet. Die Aushärtung von Dispersions- und Lösemittelklebern kann vorteilhaft durch eine UV-Behandlung vollzogen bzw. unterstützt werden.

Die Viskosität derartiger Dispersions- und Lösemittelkleber ist bei Raumtemperatur größer als 2000 mPas, sofern sie auf EVA- oder Acrylatbasis beruhen.

Nach einer bevorzugten Ausführungsform der Erfindung wird der Haftkleber mit Hilfe einer den Haftkleber führenden rotierenden Walze auf die mechanisch wirkenden Verschlusselemente aufgetragen. Hierbei ist auf der Oberfläche der Walze vorzugsweise ein im wesentlichen durchgehender Haftkleberfilm gebildet. Die mechanischen Verschlusselemente werden durch eine die eingangs erwähnte Tragschicht bildende Flachmaterialbahn und von deren Oberfläche vorstehenden hakenbildenden Vorsprüngen gebildet, wobei die Flachmaterialbahn der Walze kontinuierlich zugeführt und mit dieser in Kontakt gebracht wird.

Die Umfangsfläche der Walze kann eine Gravur aufweisen, die durch voneinander isolierte Vertiefungen gebildet ist. Die Walze kann aber auch ein nachgiebig elastisches Material umfassen. Es hat sich als besonders vorteilhaft erwiesen, wenn die Anordnung der voneinander isolierten Vertiefungen der Anordnung der hakenbildenden Vorsprünge auf der Flachmaterialbahn entspricht.

Es hat sich desweiteren als vorteilhaft erwiesen, wenn die Projektionsfläche der Vertiefungen in etwa der Größe der Projektionsfläche der hakenbildenden Vorsprünge, insbesondere der Köpfe der hakenbildenden Vorsprünge entsprechen.

Es hat sich desweiteren als besonders vorteilhaft erwiesen, wenn die Flachmaterialbahn mit den hakenbildenden Vorsprüngen der rotierenden Walze zugeführt und mit dieser derart in Kontakt gebracht wird, dass die von der Tragschicht abgewandten äußeren Oberflächen der hakenbildenden Vorsprünge den den Haftkleber tragenden Vertiefungen der Oberfläche der Walze gegenüberliegen, so dass je nach Eindringtiefe und Größe der Vertiefungen zumindest ein Teil der von der Tragschicht abgewandten Enden der hakenbildenden Vorsprünge mit Haftkleber überzogen werden.

Es kann sich dabei auch als vorteilhaft erweisen, wenn die Flachmaterialbahn über eine Gegendruckwalze gegen die den Haftkleber führende Walze geführt wird. Auf diese Weise lässt sich ein vorbestimmter Walzenspalt einstellen.

Eine weitere vorteilhafte Möglichkeit zum Auftragen des Haftklebers besteht darin, dass der Haftkleber über eine eine schlitzförmige Austragöffnung aufweisende Klebstoffauftrageinrichtung appliziert wird.

Vorzugsweise werden die mechanischen Verschlusselemente als Teil einer Flachmaterialbahn kontinuierlich unter der schlitzförmigen Austragöffnung hindurchgeführt, wobei die schlitzförmige Austragöffnung quer zur Transportrichtung der Flachmaterialbahn angeordnet ist.

Der Klebstoff kann auch über eine Vielzahl von ggf. auch gestaffelt angeordneten und voneinander beabstandeten schlitzförmigen Austragöffnungen appliziert werden, so dass eine Vielzahl diskreter Haftkleberspuren auf der Flachmaterialbahn gebildet werden. Solchenfalls entspricht der Abstand der schlitzförmigen Austragöffnungen und damit der Abstand der erzeugten Haftkleber dem Abstand der reihenbildenden hakenförmigen Vorsprünge oder einem Vielfachen dieses Abstands.

Der Haftkleber kann aber auch mittels wenigstens eines Sprühkopfs auf die mechanischen Verschlusselemente aufgetragen werden.

Eine weitere bevorzugte Möglichkeit des Haftkleberauftrags stellt der Auftrag mittels einer umdrehbaren Siebtrommel dar. Die mechanischen Verschlusselemente werden wiederum in Form einer Flachmaterialbahn kontinuierlich der Umfangsfläche der den Haftkleber freigebenden rotierenden Siebtrommel zugeführt und mit dieser in Kontakt gebracht. Wiederum entspricht das Muster des Siebes, d.h. Anzahl, Abstand und Verteilung der voneinander isolierten und mit dem Trommelinneren kommunizierenden Öffnungen der Anordnung der hakenbildenden Vorsprünge auf der Tragschicht der Flachmaterialbahn. Es kann sich wiederum als vorteilhaft erweisen, wenn die Projektionsfläche der Sieböffnungen in etwa der Größe der Projektionsfläche des freien Endes, insbesondere des Kopfs, der hakenbildenden Vorsprünge entspricht.

Vorzugsweise werden die hakenbildenden Vorsprünge derart gegenüber den Sieböffnungen ausgerichtet und mit diesen in Kontakt gebracht, dass je nach Größe der Öffnungen und Eindringtiefe der hakenbildenden Vorsprünge der Haftkleberauftrag beeinflusst werden kann.

Es hat sich insgesamt als vorteilhaft erwiesen, wenn der Haftkleber mit einem Flächengewicht von 5 bis 50 g/m² entsprechend einer Schichtdicke zwischen 5 bis 50 µm aufgetragen wird.

Es kommt aber auch ein Haftkleberauftrag im indirekten Transferverfahren in Frage, wonach der Haftkleber zunächst auf eine erste Flachmaterialbahn aufgetragen und anschließend von der ersten Flachmaterialbahn auf eine zweite Flachmaterialbahn, welche die mechanischen Verschlusselemente bildet, übertragen wird. Auf die erste Bahn wird vorzugsweise ein durchgehender Haftkleberfilm aufgetragen.

In bevorzugter Weise wird der Haftkleber bei einem Transferverfahren von einer in einem Haftkleberbad umdrehbaren Walze aufgenommen und auf eine zweite rotierende Walze übertragen und schließlich von dieser auf die Flachmaterialbahn appliziert. Die erste Walze besteht dabei vorzugsweise aus Stahl und wirkt mit einer Rakeleinrichtung derart zusammen, dass eine Haftkleberbeschichtung von maximal 10 g/m² auf der ersten Walze gebildet wird. Die mittlere Walze kann auch ein gummielastisches Walzenmaterial ggf. mit einer strukturierten Oberfläche umfassen. Es erweist sich als vorteilhaft, wenn die strukturierte Oberfläche voneinander isolierte Erhebungen aufweist, die der Anordnung der hakenbildenden Vorsprünge auf der Flachmaterialbahn entsprechen, so dass hierdurch der Haftkleberauftrag auf die Vorsprünge gesteuert werden kann.

In der Zeichnung zeigen:

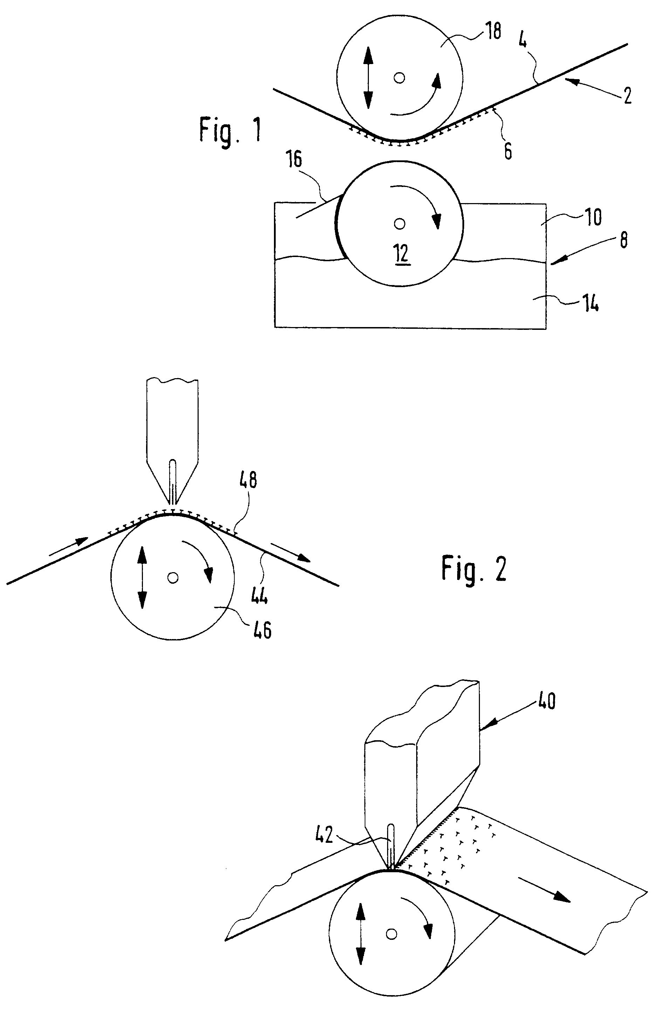

- Figuren 1 bis 6: verschiedene bevorzugte Arten des Haftkleberauftrags.

Figur 1 zeigt eine Flachmaterialbahn 2, die zur Bildung mechanischer Verschlusselemente eines Hygieneartikels weiterverarbeitet wird und eine Trägerschicht 4 und von einer Seite der Trägerschicht vorspringende hakenbildende Vorsprünge 6 aufweist, die bei dem Hygieneartikel mit nicht dargestellten Festhaltegegenmitteln zusammenwirken, welche schlaufenförmig, flauschförmig oder in sonstiger Weise derart ausgebildet sein können, dass die mechanisch wirkenden Verschlusselemente über ihre hakenförmigen Vorsprünge 6 daran mechanisch halten, insbesondere verhaken können. Mit dem Bezugszeichen 8 ist eine Haftkleberauftrageinrichtung bezeichnet und schematisch dargestellt. Sie umfasst ein Behältnis 10, welches eine erste Walze 12 derart umdrehbar lagert, dass sich die Walze 12 mit ihrer Umfangsfläche durch ein Haftkleberbad 14 bewegen kann. Die erste Walze 12 ragt in der Darstellung der Figur 1 mit einem kreissegmentförmigen Abschnitt aus dem Behältnis 10 vor. Im Bereich der Öffnung ist eine Rakeleinrichtung 16 vorgesehen, die für einen gleichmäßigen Kleberauftrag auf der Umfangsfläche der Walze 12 von z.B. 10 g/m² sorgt. Die kontinuierlich zugeführte Flachmaterialbahn 2 ist über eine heb- und senkbare Gegendruckwalze 18 geführt, die gegen die Umfangsfläche der ersten Walze 12 derart verstellt werden kann, dass die hakenbildenden Vorsprünge 6 der Flachmaterialbahn 2 in Kontakt mit der klebstoffführenden Umfangsfläche der Walze 12 gebracht werden können.

Figur 2 zeigt schematisch den Klebstoffauftrag mittels einer Auftrageinrichtung 40, die eine schlitzförmige Austragöffnung 42 umfasst. Wiederum ist eine Flachmaterialbahn 44 über eine heb- und senkbare Walze 46 geführt. Durch Einstellen des Abstands zwischen der Austragöffnung 42 und dem freien Ende bzw. Kopf der hakenbildenden Vorsprünge 48 kann die Art und Weise des Klebstoffauftrags gesteuert werden. Wenn der Abstand gegen Null geht, so wird der Haftkleber bevorzugt in die Zwischenräume zwischen den hakenbildenden Vorsprünge 48 eingepresst. Wenn der Abstand jedoch größer ist, so wird bevorzugt das freie Ende der hakenbildenden Vorsprünge 48 mit einem Haftkleberauftrag überzogen.

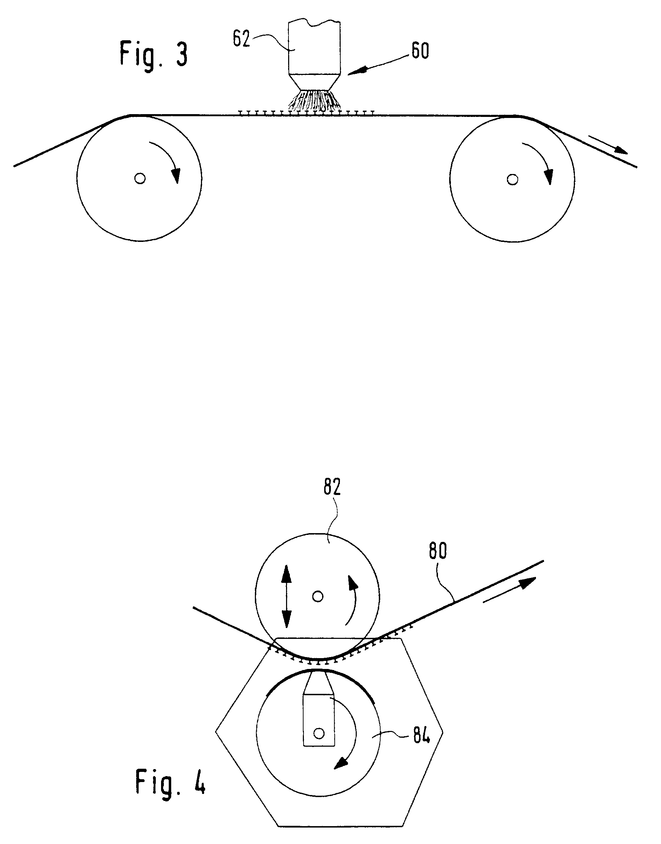

Figur 3 zeigt schematisch das Aufsprühen von Haftkleber mittels einer Sprüheinrichtung 60 mit wenigstens einem Sprühkopf 62.

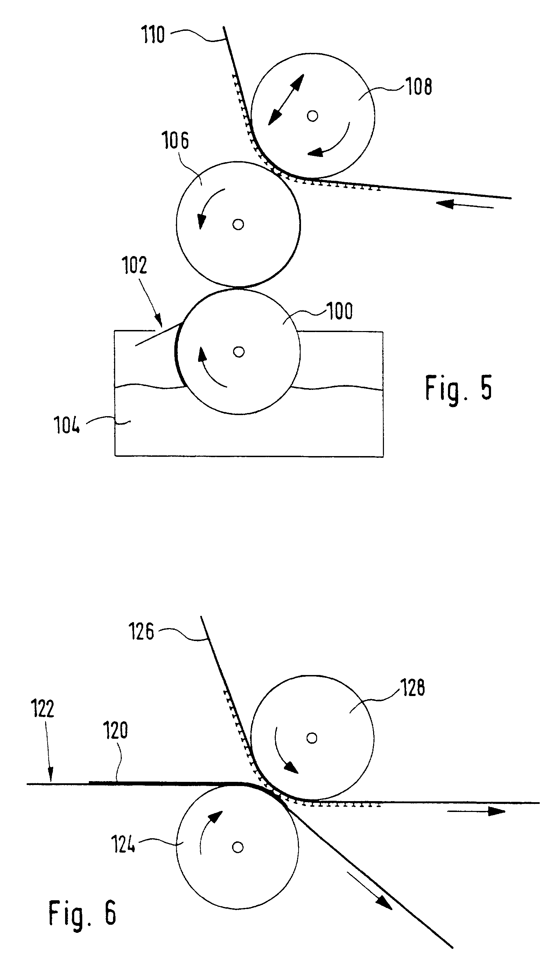

Figur 4 verdeutlicht in schematischer Darstellung das Auftragen von Haftkleber im Siebdruckverfahren. Eine die mechanischen Verschlusselemente für einen Hygieneartikel bildende Flachmaterialbahn 80 wird mittels einer Gegendruckwalze 82 in Kontakt mit einer umdrehbaren Siebtrommel 84 gebracht.

Figur 5 veranschaulicht ein indirektes Transferverfahren, wonach eine erste Aufnahmewalze 100 in Zusammenwirken mit einer Rakeleinrichtung 102 Haftkleber aus einem Haftkleberbad 104 aufnimmt und an eine eine glatte oder strukturierte Oberfläche aufweisende zweite Walze 106 übergibt.

Mittels einer dritten Gegendruckwalze 108 wird eine die mechanisch wirkenden Verschlusselemente bildende Flachmaterialbahn 110 in Kontakt mit dem Umfang der zweiten als Transferwalze wirkenden Walze 106 gebracht.

Figur 6 zeigt ein weiteres indirektes Transferverfahren, wonach ein Haftklebermaterial 120 zunächst auf eine erste als Transfermittel dienende Flachmaterialbahn 122 aufgebracht wird. Diese Flachmaterialbahn 122 wird dann über eine beheizbare Walze 124 geführt, gegen deren Umfangsfläche eine die mechanischen Verschlusselemente bildende Flachmaterialbahn 126 mittels einer Gegendruckwalze 128 abrollt. Die als Transfermittel dienende Flachmaterialbahn 122 besteht beispielsweise aus Silikonpapier. Durch die Erwärmung der beheizbaren Walze 124 wird der Haftkleber 120 auf die Flachmaterialbahn 126 übertragen.

## Patentansprüche

1. Verfahren zum Herstellen von Verschlusselementen bei saugfähigen Hygieneartikeln zum einmaligen Gebrauch, insbesondere Windeln, Inkontinenzartikeln, Windel - oder Inkontinenzhöschen oder -vorlagen, wobei die mechanisch wirkenden Verschlusselemente durch Aufbringen eines Haftschmelzklebers klebend ausgestaltet werden, **dadurch gekennzeichnet, dass** der Haftschmelzkleber bei einer Applikationstemperatur von 100 bis 180°C und bei einer Viskosität zwischen 800 und 15000 mPas aufgetragen wird.

2. Verfahren nach Anspruch 1, **dadurch gekennzeichnet, dass** die mechanisch wirkenden Verschlusselemente Teil eines Klettverschlusssystems sind und die hintergreifende hakenbildende Komponente des Klettverschlusssystems bilden.

3. Verfahren nach einem oder mehreren der vorstehenden Ansprüche, **dadurch gekennzeichnet, dass** die mechanisch wirkenden Verschlusselemente von einer Tragschicht (4) und von deren Oberfläche vorstehenden hakenbildenden Vorsprüngen (6) gebildet werden.

4. Verfahren nach einem oder mehreren der vorstehenden Ansprüche, **dadurch gekennzeichnet, dass** die hakenbildenden Vorsprünge (6) mit einem hintergreifbaren Abschnitt ausgebildet werden und einen Stamm und einen das von der Tragschicht abgewandte freie Ende bildenden Kopf aufweisen, welcher den hintergreifbaren Abschnitt bildet.

5. Verfahren nach einem oder mehreren der vorstehenden Ansprüche, **dadurch gekennzeichnet, dass** der Haftkleber auf zumindest einen Teil der Oberfläche der hakenbildenden Vorsprünge (6) aufgetragen wird.

6. Verfahren nach einem oder mehreren der vorstehenden Ansprüche, **dadurch gekennzeichnet, dass** der Haftkleber auf zumindest einen Teil der von der Tragschicht abgewandten Oberfläche des Kopfs der hakenbildenden Vorsprünge (6) aufgetragen wird.

7. Verfahren nach einem oder mehreren der vorstehenden Ansprüche, **dadurch gekennzeichnet, dass** der Haftkleber auf die Oberfläche des Stamms der hakenbildenden Vorsprünge (6) aufgetragen wird.

8. Verfahren nach einem oder mehreren der vorstehenden Ansprüche, **dadurch gekennzeichnet, dass** der Haftkleber auf zumindest einen Teil der Oberfläche der Tragschicht (4) zwischen den hakenbildenden Vorsprüngen (6) aufgetragen wird.

9. Verfahren nach einem oder mehreren der vorstehenden Ansprüche, **dadurch gekennzeichnet, dass** das Flächengewicht des aufgetragenen Haftklebers 5 bis 50 g/m² beträgt.

10. Verfahren nach einem oder mehreren der vorstehenden Ansprüche, **dadurch gekennzeichnet, dass** die Schichtdicke des aufgetragenen Haftklebers 5 bis 50 µm beträgt.

11. Verfahren nach einem oder mehreren der vorstehenden Ansprüche, **dadurch gekennzeichnet, dass** die mechanischen Verschlusselemente, bevor sie klebend ausgestaltet werden, vorbehandelt werden.

12. Verfahren nach einem oder mehreren der vorstehenden Ansprüche, **dadurch gekennzeichnet, dass** diese Vorbehandlung eine Coronabehandlung oder eine Beschichtung mit einem Haftvermittler ist.

13. Verfahren nach einem oder mehreren der vorstehenden Ansprüche, **dadurch gekennzeichnet, dass** der Haftkleber mittels einer den Haftkleber führenden rotierenden Walze (12) auf die mechanisch wirkenden Verschlusselemente aufgetragen wird.

14. Verfahren nach einem oder mehreren der vorstehenden Ansprüche, **dadurch gekennzeichnet, dass** zum Auftragen des Haftklebers ein durchgehender Haftkleberfilm auf der Oberfläche der rotierenden Walze (12) gebildet wird.

15. Verfahren nach einem oder mehreren der vorstehenden Ansprüche, **dadurch gekennzeichnet, dass** die mechanischen Verschlusselemente durch eine eine Tragschicht (4) bildende Flachmaterialbahn (2) und von deren Oberfläche vorstehenden hakenbildenden Vorsprüngen (6) gebildet werden und dass die Flachmaterialbahn (2) der Walze (12) kontinuierlich zugeführt und mit der Umfangsfläche der Walze in Kontakt gebracht wird.

16. Verfahren nach einem oder mehreren der vorstehenden Ansprüche, **dadurch gekennzeichnet, dass** eine Walze mit einer auf ihrer Umfangsfläche vorgesehenen Gravur verwendet wird.

17. Verfahren nach einem oder mehreren der vorstehenden Ansprüche, **dadurch gekennzeichnet, dass** die Walze ein nachgiebig elastisches Material umfasst.

18. Verfahren nach einem oder mehreren der vorstehenden Ansprüche, **dadurch gekennzeichnet, dass** eine Walze mit einer Gravur, die von musterförmig angeordneten Vertiefungen gebildet ist, verwendet wird.

19. Verfahren nach einem oder mehreren der vorstehenden Ansprüche, **dadurch gekennzeichnet, dass** eine Walze verwendet wird, bei der die Anordnung der voneinander isolierten Vertiefungen der Anordnung der hakenbildenden Vorsprünge auf der Flachmaterialbahn entspricht.

20. Verfahren nach einem oder mehreren der vorstehenden Ansprüche, **dadurch gekennzeichnet, dass** die Projektionsfläche der Vertiefungen in etwa der Größe der hakenbildenden Vorsprünge, insbesondere der Köpfe der hakenbildenden Vorsprünge, entspricht.

21. Verfahren nach einem oder mehreren der vorstehenden Ansprüche, **dadurch gekennzeichnet, dass** die Flachmaterialbahn mit den hakenbildenden Vorsprüngen der rotierenden Walze zugeführt und mit dieser derart in Kontakt gebracht wird, dass die von der Tragschicht abgewandten äußeren Oberflächen der hakenbildenden Vorsprünge den den Haftkleber tragenden Vertiefungen der Oberfläche der Walze gegenüberliegen, so dass je nach Eindringtiefe und Größe der Vertiefungen zumindest ein Teil der von der Tragschicht abgewandten Enden der hakenbildenden Vorsprünge mit Haftkleber überzogen werden.

22. Verfahren nach einem oder mehreren der vorstehenden Ansprüche, **dadurch gekennzeichnet, dass** die Flachmaterialbahn über eine Gegendruckwalze gegen die den Haftkleber führende Walze geführt wird.

23. Verfahren nach einem oder mehreren der vorstehenden Ansprüche, **dadurch gekennzeichnet, dass** der Haftkleber über eine eine schlitzförmige Austragöffnung aufweisende Klebstoffauftrageinrichtung appliziert wird.

24. Verfahren nach einem oder mehreren der vorstehenden Ansprüche, **dadurch gekennzeichnet, dass** die mechanischen Verschlusselemente als Teil einer Flachmaterialbahn kontinuierlich unter der schlitzförmigen Austragöffnung hindurchgeführt werden, wobei die schlitzförmige Austragöffnung quer zur Transportrichtung der Flachmaterialbahn angeordnet wird.

25. Verfahren nach einem oder mehreren der vorstehenden Ansprüche, **dadurch gekennzeichnet, dass** der Klebstoffauftrag durch eine Vielzahl von insbesondere gestaffelt angeordneten und voneinander beabstandeten schlitzförmigen Austragöffnungen durchgeführt wird.

26. Verfahren nach einem oder mehreren der vorstehenden Ansprüche, **dadurch gekennzeichnet, dass** der Abstand der schlitzförmigen Austragöffnungen und damit der Abstand der hierdurch erzeugten Leimspuren dem Abstand der Reihen bildenden hakenförmigen Vorsprünge oder einem Vielfachen desselben entspricht.

27. Verfahren nach einem oder mehreren der vorstehenden Ansprüche, **dadurch gekennzeichnet, dass** der Haftkleber mittels wenigstens eines Sprühkopfs auf die mechanischen Verschlusselemente aufgetragen wird.

28. Verfahren nach wenigstens einem der vorstehenden Ansprüche, **dadurch gekennzeichnet, dass** der Haftkleber mittels einer umdrehbaren Siebtrommel aufgetragen wird.

29. Verfahren nach einem oder mehreren der vorstehenden Ansprüche, **dadurch gekennzeichnet, dass** die mechanischen Verschlusselemente in Form einer Flachmaterialbahn kontinuierlich der Umfangsfläche der den Haftkleber freigebenden rotierenden Siebtrommel zugeführt und mit dieser in Kontakt gebracht werden.

30. Verfahren nach einem oder mehreren der vorstehenden Ansprüche, **dadurch gekennzeichnet, dass** ein Sieb verwendet wird, bei dem Anzahl, Abstand und Verteilung der voneinander isolierten und mit dem Trommelinneren kommunizierenden Öffnungen der Anordnung der hakenbildenden Vorsprünge auf der Tragschicht der Flachmaterialbahn entspricht.

31. Verfahren nach einem oder mehreren der vorstehenden Ansprüche, **dadurch gekennzeichnet, dass** die Projektionsfläche der Sieböffnungen in etwa der Größe der Projektionsflächen des freien Endes der hakenbildenden Vorsprünge entspricht.

32. Verfahren nach einem oder mehreren der vorstehenden Ansprüche, **dadurch gekennzeichnet, dass** die hakenbildenden Vorsprünge derart gegenüber den Sieböffnungen ausgerichtet und mit diesen in Kontakt gebracht werden, dass je nach Größe der Öffnungen und Eindringtiefe der hakenbildenden Vorsprünge der Haftkleberauftrag beeinflusst werden kann.

33. Verfahren nach einem oder mehreren der vorstehenden Ansprüche, **dadurch gekennzeichnet, dass** der Haftkleberauftrag im indirekten Transferverfahren durchgeführt wird.

34. Verfahren nach einem oder mehreren der vorstehenden Ansprüche, **dadurch gekennzeichnet, dass** der Haftkleber zunächst auf eine erste Flachmaterialbahn aufgetragen und anschließend von der ersten Flachmaterialbahn auf eine zweite Flachmaterialbahn, welche die mechanischen Verschlusselemente bildet, übertragen wird.

35. Verfahren nach einem oder mehreren der vorstehenden Ansprüche, **dadurch gekennzeichnet, dass** auf die erste Bahn ein vorzugsweise durchgehender Haftkleberfilm aufgetragen wird.

36. Verfahren nach einem oder mehreren der vorstehenden Ansprüche, **dadurch gekennzeichnet, dass** der Haftkleber von einer in einem Haftkleberbad umdrehbaren Walze aufgenommen und auf eine zweite rotierende Walze übertragen und schließlich von dieser auf die Flachmaterialbahn appliziert wird.

37. Verfahren nach einem oder mehreren der vorstehenden Ansprüche, **dadurch gekennzeichnet, dass** die zweite Walze eine strukturierte Oberfläche aufweist.

38. Verfahren nach einem oder mehreren der vorstehenden Ansprüche, **dadurch gekennzeichnet, dass** die strukturierte Oberfläche durch voneinander isolierte Erhebungen gebildet ist.

## Claims

1. Method for producing fastening elements in absorbent disposable sanitary articles, in particular nappies, incontinence articles, nappy or incontinence pants or inserts, the mechanically acting fastening elements being designed in an adhesive manner by applying a hot-melt-type adhesive, **characterised in that** the hot-melt-type adhesive is applied at an application temperature of 100 to 180°C and at a viscosity between 800 and 15,000 mPas.

2. Method according to claim 1, **characterised in that** the mechanically acting fastening elements are part of a burr-type fastening system and form the engaging hook-forming components of the burr-type fastening system.

3. Method according to any one or more of the preceding claims, **characterised in that** the mechanically acting fastening elements are formed by a carrier layer (4) and by hook-forming projections (6) projecting from the surface thereof.

4. Method according to any one or more of the preceding claims, **characterised in that** the hook-forming projections (6) are formed with an engageable portion and comprise a stem and a head forming the free end remote from the carrier layer, which head forms the engageable portion.

5. Method according to any one or more of the preceding claims, **characterised in that** the adhesive is applied to at least a portion of the surface of the hook-forming projections (6).

6. Method according to any one or more of the preceding claims, **characterised in that** the adhesive is applied to at least a portion of the surface of the head of the hook-forming projections (6) remote from the carrier layer.

7. Method according to any one or more of the preceding claims, **characterised in that** the adhesive is applied to the surface of the stem of the hook-forming projections (6).

8. Method according to any one or more of the preceding claims, **characterised in that** the adhesive is applied to at least a portion of the surface of the carrier layer (4) between the hook-forming projections (6).

9. Method according to any one or more of the preceding claims, **characterised in that** the mass per unit area of the applied adhesive is 5 to 50 g/m².

10. Method according to any one or more of the preceding claims, **characterised in that** the layer thickness of the applied adhesive is 5 to 50 µm.

11. Method according to any one or more of the preceding claims, **characterised in that** the mechanical fastening elements are pre-treated before they are made adhesive.

12. Method according to any one or more of the preceding claims, **characterised in that** this pre-treatment is a corona treatment or a coating with a bonding agent.

13. Method according to any one or more of the preceding claims, **characterised in that** the adhesive is applied by means of a rotating roller (12) guiding the adhesive to the mechanically acting fastening elements.

14. Method according to any one or more of the preceding claims, **characterised in that** a continuous film of adhesive is formed on the surface of the rotating roller (12) to apply the adhesive.

15. Method according to any one or more of the preceding claims, **characterised in that** the mechanical fastening elements are formed by a web of flat material (2) forming a carrier layer (4) and by hook-forming projections (6) projecting from the surface thereof, and **in that** the web of flat material (2) is continuously supplied to the roller (12) and is brought into contact with the peripheral face of the roller.

16. Method according to any one or more of the preceding claims, **characterised in that** a roller with an engraving provided on its peripheral face is used.

17. Method according to any one or more of the preceding claims, **characterised in that** the roller comprises a flexibly elastic material.

18. Method according to any one or more of the preceding claims, **characterised in that** a roller with an engraving formed by indents arranged in the manner of a pattern is used.

19. Method according to any one or more of the preceding claims, **characterised in that** a roller is used in which the arrangement of indents which are isolated from one another corresponds to the arrangement of the hook-forming projections on the web of flat material.

20. Method according to any one or more of the preceding claims, **characterised in that** the projection face of the indents corresponds approximately to the size of the hook-forming projections, in particular the heads of the hook-forming projections.

21. Method according to any one or more of the preceding claims, **characterised in that** the web of flat material with the hook-forming projections is supplied to the rotating roller and is brought into contact therewith in such a way that the external surfaces of the hook-forming projections remote from the carrier layer oppose the indents of the surface of the roller carrying the adhesive, so at least a portion of the ends of the hook-forming projections remote from the carrier layer are coated with adhesive, depending on the penetration depth and size of the indents.

22. Method according to any one or more of the preceding claims, **characterised in that** the web of flat material is guided via a counter pressure roller toward the roller carrying the adhesive.

23. Method according to any one or more of the preceding claims, **characterised in that** the adhesive is applied via an adhesive application device comprising a slot-shaped discharge aperture.

24. Method according to any one or more of the preceding claims, **characterised in that** the mechanical fastening elements are continuously guided under the slot-shaped discharge aperture as part of a web of flat material, the slot-shaped discharge aperture being arranged transversely to the conveying direction of the web of flat material.

25. Method according to any one or more of the preceding claims, **characterised in that** the adhesive is applied by a plurality of, in particular, staggered and mutually spaced apart slot-shaped discharge apertures.

26. Method according to any one or more of the preceding claims, **characterised in that** the spacing of the slot-shaped discharge apertures, and therefore the spacing of the glue tracks produced thereby, corresponds to the spacing of the hook-shaped projections forming the rows or a multiple thereof.

27. Method according to any one or more of the preceding claims, **characterised in that** the adhesive is applied by means of at least one spray head onto the mechanical fastening elements.

28. Method according to at least any one of the preceding claims, **characterised in that** the adhesive is applied by means of a rotatable perforated drum.

29. Method according to any one or more of the preceding claims, **characterised in that** the mechanical fastening elements are continuously supplied in the form of a web of flat material to the peripheral face of the rotating perforated drum releasing the adhesive and are brought into contact therewith.

30. Method according to any one or more of the preceding claims, **characterised in that** a screen is used in which the number, spacing and distribution of apertures isolated from one another and communicating with the interior of the drum corresponds to the arrangement of the hook-forming projections on the carrier layer of the web of flat material.

31. Method according to any one or more of the preceding claims, **characterised in that** the projection face of the screen apertures corresponds approximately to the size of the projection faces of the free end of the hook-forming projections.

32. Method according to any one or more of the preceding claims, **characterised in that** the hook-forming projections are aligned with respect to the screen apertures and are brought into contact therewith in such a way that the application of adhesive can be influenced depending on the size of the apertures and penetration depth of the hook-forming projections.

33. Method according to any one or more of the preceding claims, **characterised in that** the adhesive is applied using the indirect transfer method.

34. Method according to any one or more of the preceding claims, **characterised in that** the adhesive is initially applied to a first web of flat material and is subsequently transferred from the first web of flat material to a second web of flat material forming the mechanical fastening element.

35. Method according to any one or more of the preceding claims, **characterised in that** a preferably continuous film of adhesive is applied to the first web.

36. Method according to any one or more of the preceding claims, **characterised in that** the adhesive is taken up by a roller which is rotatable in a bath of adhesive and is transferred to a second rotating roller and is finally applied therefrom to the web of flat material.

37. Method according to any one or more of the preceding claims, **characterised in that** the second roller has a structured surface.

38. Method according to any one or more of the preceding claims, **characterised in that** the structured surface is formed by elevations which are isolated from one another.

## Revendications

1. Procédé de fabrication d'éléments de fermeture destinés à des articles d'hygiène absorbants à usage unique, en particulier des couches, des articles pour incontinents, des couches-culottes ou des protections pour incontinents, dans lequel les éléments de fermeture à action mécanique sont formés pour coller par l'application d'une colle thermo-adhésive, **caractérisé en ce que** la colle thermo-adhésive est appliquée à une température d'application comprise entre 100 et 180 °C et une viscosité comprise entre 800 et 1 500 mPa.s.

2. Procédé selon la revendication 1, **caractérisé en ce que** les éléments de fermeture à action mécanique sont une partie d'un système de fermeture auto-accrochant et forment les composants formant des crochets à accrochage arrière du système de fermeture auto-accrochant.

3. Procédé selon une ou plusieurs des revendications précédentes, **caractérisé en ce que** les éléments de fermeture à action mécanique sont formés d'une couche de support (4) et de saillies formant des crochets (6) qui dépassent sur sa surface.

4. Procédé selon la revendication précédente, **caractérisé en ce que** les saillies formant des crochets (6) sont conçues avec un segment à accrochage par l'arrière et présentent une tige et une tête formant l'extrémité libre orientée à l'opposé de la couche de support, laquelle forme le segment à accrochage par l'arrière.

5. Procédé selon la revendication 3 et l'une des revendications 1, 2 ou 4, **caractérisé en ce que** la colle adhésive est appliquée sur au moins une partie de la surface des saillies formant des crochets (6).

6. Procédé selon l'une des revendications 3 ou 4, **caractérisé en ce que** la colle adhésive est appliquée sur au moins une partie de la surface de la tête, orientée à l'opposé de la couche de support, des saillies formant des crochets (6).

7. Procédé selon une ou plusieurs des revendications 3 à 6, **caractérisé en ce que** la colle adhésive est appliquée sur la surface de la tige des saillies formant des crochets (6).

8. Procédé selon une ou plusieurs des revendications 3 à 7, **caractérisé en ce que** la colle adhésive est appliquée sur au moins une partie de la surface de la couche de support (4) entre les saillies formant des crochets (6).

9. Procédé selon une ou plusieurs des revendications précédentes, **caractérisé en ce que** le grammage de la colle adhésive appliquée est compris entre 5 et 50 g/m².

10. Procédé selon une ou plusieurs des revendications précédentes, **caractérisé en ce que** l'épaisseur de couche de la colle adhésive appliquée est comprise entre 5 et 50 µm.

11. Procédé selon une ou plusieurs des revendications précédentes, **caractérisé en ce que** les éléments de fermeture mécaniques sont pré-traités avant d'être formés de façon à coller.

12. Procédé selon une ou plusieurs des revendications précédentes, **caractérisé en ce que** ce pré-traitement est un traitement corona ou un revêtement avec un agent adhésif.

13. Procédé selon une ou plusieurs des revendications précédentes, **caractérisé en ce que** la colle adhésive est appliquée sur les éléments de fermeture à action mécanique au moyen d'un cylindre rotatif (12) guidant la colle adhésive.

14. Procédé selon une ou plusieurs des revendications précédentes, **caractérisé en ce que**, pour appliquer la colle adhésive, on forme un film de colle adhésive continu sur la surface du cylindre rotatif (12).

15. Procédé selon une ou plusieurs des revendications précédentes, **caractérisé en ce que** les éléments de fermeture mécaniques sont formés par une bande de matériau plane (2) formant une couche de support (4) et par des saillies formant des crochets (6) dépassant sur sa surface, et **en ce que** la bande de matériau plane (2) est amenée en continu au cylindre (12) et mise en contact avec la surface périphérique du cylindre.

16. Procédé selon une ou plusieurs des revendications précédentes, **caractérisé en ce que** l'on utilise un cylindre avec une gravure prévue sur sa surface périphérique.

17. Procédé selon la revendication précédente, **caractérisé en ce que** le cylindre comprend un matériau élastique flexible.

18. Procédé selon l'une des revendications 16 ou 17, **caractérisé en ce que** l'on utilise un cylindre avec une gravure qui est formée de creux disposés pour former un motif.

19. Procédé selon l'une des revendications 16 à 18, **caractérisé en ce que** l'on utilise un cylindre dans lequel la disposition des creux isolés les uns des autres correspond à la disposition des saillies formant des crochets sur la bande de matériau plane.

20. Procédé selon l'une des revendications 3 à 19, **caractérisé en ce que** la surface de projection des creux correspond approximativement à la taille des saillies formant des crochets, en particulier des têtes des saillies formant des crochets.

21. Procédé selon l'une des revendications 16 à 20, **caractérisé en ce que** la bande de matériau plane avec les saillies formant des crochets est amenée au cylindre rotatif et mise en contact avec celui-ci de façon telle que les surfaces externes des saillies formant des crochets, orientées à l'opposé de la couche de support, font face aux creux portant la colle adhésive de la surface du cylindre, de façon telle qu'en fonction de la profondeur de pénétration et de la taille des creux, au moins une partie des extrémités des saillies formant des crochets, orientées à l'opposé de la couche de support, est revêtue de colle adhésive.

22. Procédé selon l'une des revendications 15 à 21, **caractérisé en ce que** la bande de matériau plane est amenée par un cylindre de contre-pression contre le cylindre guidant la colle adhésive.

23. Procédé selon une ou plusieurs des revendications précédentes, **caractérisé en ce que** la colle adhésive est appliquée par un dispositif d'application de colle présentant des ouvertures d'enduction en forme de rainures.

24. Procédé selon une ou plusieurs des revendications précédentes, **caractérisé en ce que** les éléments de fermeture mécanique en tant que partie d'une bande de matériau plane sont amenés en continu sous l'ouverture d'enduction en forme de rainure, l'ouverture d'enduction en forme de rainure étant disposée transversalement à la direction de transport de la bande de matériau plane.

25. Procédé selon une ou plusieurs des revendications précédentes, **caractérisé en ce que** l'application de colle est réalisée par un certain nombre d'ouvertures d'enduction en forme de rainure disposées en particulier de façon décalée et écartées les unes des autres.

26. Procédé selon la revendication précédente, **caractérisé en ce que** l'écart entre les ouvertures d'enduction en forme de rainures et par conséquent l'écart entre les traces de colle ainsi créées correspond à l'écart entre les saillies en forme de crochets formant des rangées ou à un multiple de celui-ci.

27. Procédé selon une ou plusieurs des revendications précédentes, **caractérisé en ce que** la colle adhésive est appliquée sur les éléments de fermeture mécanique au moyen d'au moins une tête de pulvérisation.

28. Procédé selon au moins une des revendications précédentes, **caractérisé en ce que** la colle adhésive est appliquée au moyen d'un tambour cribleur rotatif.

29. Procédé selon une ou plusieurs des revendications précédentes, **caractérisé en ce que** les éléments de fermeture mécanique sont amenés en continu sous la forme d'une bande de matériau plane à la surface périphérique du tambour cribleur rotatif libérant la colle adhésive et mis en contact avec celle-ci.

30. Procédé selon une ou plusieurs des revendications précédentes, **caractérisé en ce que** l'on utilise un crible dans lequel le nombre, l'écart et la répartition des ouvertures isolées les unes des autres et communiquant avec l'intérieur du tambour correspondent à la disposition des saillies formant des crochets sur la couche de support de la bande de matériau plane.

31. Procédé selon une ou plusieurs des revendications précédentes, **caractérisé en ce que** la surface de projection des ouvertures de crible correspond approximativement à la taille des surfaces de projection de l'extrémité libre des saillies formant des crochets.

32. Procédé selon l'une des revendications 3 à 31, **caractérisé en ce que** les saillies formant des crochets sont exécutées par rapport aux ouvertures de crible et mises en contact avec celles-ci de façon telle que l'application de colle adhésive peut être influencée en fonction de la taille des ouvertures et de la profondeur de pénétration des saillies formant des crochets.

33. Procédé selon une ou plusieurs des revendications précédentes, **caractérisé en ce que** l'application de colle adhésive est réalisée au cours d'un procédé de transfert indirect.

34. Procédé selon une ou plusieurs des revendications précédentes, **caractérisé en ce que** la colle adhésive est d'abord appliquée sur une première bande de matériau plane, puis transférée depuis la première bande de matériau plane sur une seconde bande de matériau plane qui forme les éléments de fermeture mécanique.

35. Procédé selon une ou plusieurs des revendications précédentes, **caractérisé en ce que** l'on applique un film de colle adhésive de préférence continu sur la première bande.

36. Procédé selon une ou plusieurs des revendications précédentes, **caractérisé en ce que** la colle adhésive est absorbée par un cylindre pouvant tourner dans un bain de colle adhésive et transférée sur un second cylindre rotatif, puis appliquée depuis celui-ci sur la bande de matériau plane.

37. Procédé selon une ou plusieurs des revendications précédentes, **caractérisé en ce que** le second cylindre présente une surface structurée.

38. Procédé selon une ou plusieurs des revendications précédentes, **caractérisé en ce que** la surface structurée est formée par des élévations isolées les unes des autres.
